# EUROPEAN PATENT SPECIFICATION

(11) **EP 0 616 522 B1**
(45) Date of publication and mention of the grant of the patent: **29.05.1996**
(21) Application number: 93900049.3
(22) Date of filing: 10.12.1992
(51) Int. Cl.: A61K 7/42

(54) **SUNSCREEN COMPOSITIONS**
Sonnenschutzzusammensetzung
COMPOSITIONS DE PROTECTION SOLAIRE

(30) Priority: 12.12.1991 GB 9126440
(43) Date of publication of application: 28.09.1994
(73) Proprietor: The Boots Company PLC, Nottingham Nottinghamshire NG2 3AA (GB)
(72) Inventor: FARDELL, Nicola Anne, Nottingham NG2 3AA (GB); NUTHALL, Trevor G., Nottingham NG2 3AA (GB); STAMMERS, Arija Margaret, Nottingham NG2 3AA (GB)
(74) Representative: Kirk, Martin John
(86) International application number: EP9202897
(87) International publication number: WO9311742

(56) References cited:
- EP-A- 0 433 086
- EP-A- 0 456 458
- WO-A-90/06103
- GB-A- 2 243 780
- PATENT ABSTRACTS OF JAPAN vol. 11, no. 267 (C-443)(2714) 28 August 1987
- DATABASE WPIL Week 9040, Derwent Publications Ltd., London, GB; AN 90-301252
- PATENT ABSTRACTS OF JAPAN vol. 11, no. 176 (C-426)(2623) 5 June 1987

## Description

The present invention relates to the use of iron oxide particles in sunscreen compositions. The term "sunscreen" is used herein to encompass tanning lotions, sunscreens and sunblockers which are intended for use on the body to provide protection against the sun's rays or other UV sources.

The use of titanium dioxide as a sunscreening agent is well known. The amount of titanium dioxide present in a sunscreen composition depends on the use for which the composition is intended. Amounts as low as 1% may be sufficient in the so-called suntanning products which are not intended to prevent the sun's rays reaching the skin whereas the so-called sunblocks which are intended to prevent substantially all of the sun's rays reaching the skin may require levels of 15 to 20%. It has been found that when the amount of titanium dioxide in the sunscreen composition exceeds 5% the composition exhibits a blue-white pearl effect which is aesthetically unattractive and limits the amount of titanium dioxide which can be incorporated into a sunscreen composition which is acceptable to the user.

Use of zinc oxide as a sunscreening agent is also well known, either on its own or combined with titanium dioxide.

Hereinafter, throughout the specification and claims the term 'metal oxide' is defined as a metal oxide consisting of titanium dioxide only, zinc oxide only, or a mixture of both these oxides. In preferred sunscreen compositions of the present invention the metal oxide is titanium dioxide only.

Pigment grade iron oxides of coarse particle size have been used in sunscreen compositions as tinting agents, typically at a level of 5% w/w, to mask the blue-white pearl effect. However, such compositions suffer from the disadvantage that the iron oxide tinting agent rubs off in use and may cause unacceptable discolouration or staining of clothing.

Surprisingly we have found that the use of iron oxide of a particular particle size may be used in sunscreen formulations at comparatively low levels therefore minimising unacceptable rub off yet permitting the use of comparatively high levels of titanium dioxide without the disadvantage of an unacceptable blue-white pearl.

British Patent Application 2,243,780A (Unilever) and European Patent Application 456458 (Unilever) describe cosmetic compositions comprising microfine titanium dioxide (particle size 1 to 100 nm) as an inorganic sunscreen. Both these documents contain a passing reference to the optional use of iron oxide of particle size 1 to 300 nm as one of many optional additional inorganic sunscreens. There is no teaching that it would be advantageous to select small sized iron oxide in small amounts or that such amounts would be particularly effective as a sunscreen agent. GB 2,243,780 suggests that the iron oxide might be present up to 20% by weight, preferably 1 to 10% by weight of the composition, which teaches away from the selection (as in compositions of the present invention) of iron oxide in amounts less than 1%.

Patent abstracts of Japan Vol 11, no. 267 (C-443) (2714), which abstracts Japanese Patent Application 62-067014 (Sunstar), describes a cosmetic composition comprising 3 to 30% titanium dioxide (5 to 75 nm particle size) with 0.2 to 10% iron oxide (particle size 10 to 50 nm minor axis, 50 to 200 nm major axis) as a sunscreen and colouring agent. This grade of iron oxide is much larger (up to 100 times in size) than the iron oxide particles used in the present invention, and the Sunstar composition would be subject to the disadvantage of rub off of the iron oxide as discussed above.

Derwent WPIL abstract AN 90-301252 of Japanese Patent Application JP 2-212414 (Nonomura) describes a dispersion of surface treated powders comprising one or more of titanium dioxide, zinc oxide, or iron oxide of grain size 1 nm to 3 µm in plastic, as an ingredient for use in cosmetic compositions. The extremely broad range of particle size disclosed, does not teach the particular advantages in selecting iron oxide with a small particle size.

The present invention provides a sunscreen composition comprising metal oxide in an amount from 0.1% to 30% by weight of the total composition, the metal oxide having a mean particle size of less than 100 nm; and iron oxide in an amount from 0.01% to 1% by weight of the total composition, the iron oxide having a mean particle size from 0.1nm to 10nm.

Preferably the composition comprises metal oxide particles having a mean particle size from 5nm to 50nm and more preferably from 15nm to 30nm.

Sunscreen compositions have recently become available in which the mean particle size of titanium dioxide particles lies in the range 1 to 100nm. Titanium dioxide of the above mean particle size is usually referred to as "microfine". A suitable grade of titanium dioxide is available from Degussa under the trade designation P25 and from Tayca Corporation under the trade designation MT150W, MT600B or MT500B.

Preferably a sunscreen composition of the present invention comprises metal oxide in an amount from 0.5% to 20%, more preferably from 1% to 15% and most preferably from 4% to 12% by weight of the total composition.

Preferably a sunscreen composition of the present invention comprises iron oxide in an amount from 0.02% to 0.5%, more preferably from 0.05% to 0.25% and most preferably 0.111% by weight of the total composition.

Preferably the iron oxide particles have a mean particle size from 1nm to 5nm.

In a preferred form of the present invention the iron oxide comprises iron (III) oxide. One particularly preferred iron (III) oxide is available from Mach I Inc. under the trade designation Super-Fine Iron Oxide.

Preferably the particles of iron oxide or metal oxide or both can be substantially coated with a phospholipid, such as lecithin. This helps reduce agglomeration of the particles. Sunscreen compositions comprising phospholipids are described in the applicant's international patent application WO 90/06103 the disclosures of which are hereby incorporated by reference.

In order to substantially reduce or prevent the discoloration of the metal oxide that may occur during storage of sunscreen compositions, the compositions of the present invention may further comprise from 0.025% to 30% by weight of the composition of phosphate anions. Sunscreen compositions containing phosphates are described in the applicant's international patent application WO90/09777, the disclosures of which are hereby incorporated by reference.

Sunscreen compositions of the present invention may further comprise a water-in-oil emulsion which comprises from 5% to 70% by weight of an oil phase, from 1% to 15% by weight of an emulsifier, and at least 20% by weight of an aqueous phase.

Sunscreen compositions of the present invention may also further comprise an oil-in-water emulsion which comprises from 5% to 60% by weight of an oil phase, from 1% to 20% by weight of an emulsifier and at least 35% by weight of an aqueous phase.

Other sunscreening agents may be incorporated into the sunscreen compositions of the present invention. Examples of suitable further sunscreening agents include a) p-aminobenzoic acids, esters and derivatives (for example, 2-ethylhexyl p-dimethylaminobenzoate, or the octyl ester of p-aminobenzoic acid), b) methoxycinnamate esters [for example, 2-ethylhexyl p-methoxycinnamate, 2-ethoxyethyl p-methoxycinnamate or α,β-di-(p-methoxycinnamoyl)-α'-(2-ethylhexanoyl)-glycerin], c) benzophenones (for example oxybenzone), d) dibenzoylmethanes and e) salicylate esters. Any additional sunscreening agent is present in an amount from 0.1% to 10% by weight of the composition.

The oil phase of the water-in-oil and oil-in-water emulsions of the present invention may comprise a) hydrocarbon oils such as paraffin or mineral oils, b) waxes such as beeswax or paraffin wax, c) natural oils such as sunflower oil, apricot kernel oil, shea butter or jojoba oil, d) silicone oils such as dimethicone, cyclomethicone or cetyldimethicone, e) fatty acid esters such as isopropyl palmitate or isopropyl myristate, f) fatty alcohols such as cetyl alcohol or stearyl alcohol, or g) mixtures thereof. In preferred water-in-oil compositions of the present invention, the oil phase comprises from 5% to 60% by weight of the composition. In preferred oil-in-water compositions of the present invention, the oil phase comprises from 10% to 30% by weight of the composition.

The emulsifiers used may be any emulsifiers known in the art for use in water-in-oil or oil-in-water emulsions. It has been found that particularly effective water-in-oil sunscreen compositions can be prepared by using an emulsifier or mixture of emulsifiers selected from:
a) ethoxylated fatty alcohols, for example the emulsifier available commercially under the trade name Brij (ICI)
b) ethoxylated fatty acids and their esters such as ethoxylated stearates, for example the emulsifier available commercially under the trade name Myrj (ICI)
c) sorbitan esters, such as the emulsifier available commercially under the trade name Span (ICI)
d) ethoxylated sorbitan esters, such as the emulsifier available commercially under the trade name Tween (ICI)
e) ethoxylated mono-, di- and tri-glycerides, for example the emulsifier available commercially under the trade name Arlatone (ICI)
f) silicon emulsifiers such as silicone polyols, available commercially for example under the trade name Abil WS08
g) anionic emulsifiers for example cetylstearyl sulphates available under the trade name Dehydag and anionic esters of mono and diglyercides available under the trade name Grindtek
h) cationic emulsifiers for example dimethyldistearylammonium chloride available under the trade name Genamin DSAC.

Particularly effective oil-in-water sunscreen compositions can be prepared by using an emulsifier or mixture of emulsifiers selected from known cosmetically acceptable emulsifiers which include a) fatty acid soaps such as potassium stearate, b) ethoxylated fatty alcohols, for example the emulsifier available commercially under the trade name Brij (ICI), c) sorbitan esters, for example the emulsifier available commercially under the trade name Span (ICI), d) ethoxylated sorbitan esters, for example the emulsifier available commercially under the trade name Tween (ICI), e) ethoxylated fatty acid esters such as ethoxylated stearates, for example the emulsifier available commercially under the trade name Myrj (ICI), f) ethoxylated mono-, di-, and tri-glycerides, for example the emulsifier available commercially under the trade name Arlatone (ICI), or g) mixtures thereof. The amount of emulsifier present in the oil-in-water compositions of the present invention is preferably from 1% to 10% by weight of the composition.

The compositions of the present invention may additionally comprise other components which will be well known to those skilled in the art for example emollients such as isopropyl myristate or a triglyceride of a fatty acid (e.g. lauric triglyceride or capric/caprylic triglyceride), moisturisers such as D-panthenol, humectants such as glycerin or 1,3-butylene-glycol, antioxidants such as (D,L)-α-tocophenyl-acetate or butylated hydroxytoluene, emulsion stabilising salts such as sodium chloride, sodium citrate or magnesium sulphate, film formers to assist spreading on the surface of the skin such as alkylated polyvinylpyrrolidone, preservatives such as bronopol, sodium dehydroacetate, polyhexamethylenebiguanide hydrochloride, isothiazolone or diazolidinylurea, perfumes and colouring.

The compositions of the present invention described hereinbefore provide adequate protection against both UVA and UVB radiation in a composition which is aesthetically pleasing to the user. Preferred compositions are substantially transparent when applied to the skin.

The opacity or whiteness of compositions may be estimated by trained observers by subjective visual analysis of the relative transparency of the composition spread onto skin. However, a quantitative assessment of the opacity or whiteness of compositions may be obtained as follows.

A circular area of diameter 31mm was marked on the forearm of a volunteer and the surface reflectance of that area measured with a Minolta chromometer. 10µl of product was then applied to the test site and spread evenly with a glass rod. The product was left to dry for 2 to 3 minutes and then the surface reflectance of the area measured again. Each product was tested on several volunteers. The difference between the mean surface reflectance (L-value) before and after product application gives a quantitative indication of the whiteness of the product.

Products giving a mean L-value increase of greater than 5.5 were considered to be cosmetically unacceptable. Preferred compositions according to the invention had a mean L-value increase of less than 5.0.

The efficacy of compositions of the present invention as sunscreens was demonstrated as follows. The Simpson's ratio of UVA to UVB absorption was measured by the method described in full by Diffey and Robson in J. Soc. Cosmet. Chem., 40, 127-133 (1989) but which is summarised below.

Adhesive tape commercially available from 3M-Company, USA, under the trade name Transpore® was used as the medium to which the product was applied. This tape is UV-transparent and has a textured surface which has been found to distribute sunscreen compositions in a way similar to the uneven topography of human skin. A piece of tape (75mm x 65mm) was placed over the input slit of an f 3.4 grating monochromator (Applied Photophysics Ltd, UK), attached to a photomultiplier detector, and illuminated with "white" light from a 75 W xenon arc source. The intensity of the radiation transmitted through the tape was measured by recording the photocurrent in 5nm steps from 290-400nm.

100µl of sunscreen (2.0mg/cm²) was evenly distributed onto the tape surface, the tape replaced over the monochromator input slit and transmission measurements were repeated. For each product the transmittance was determined at 12 separate subsites within the 77 x 65mm application site. The Simpson's ratio of absorption in the range 320-400nm (UVA) and absorption in the range 290-320nm (UVB) was then calculated from the mean measurement at each wavelength.

The invention will now be illustrated by the following non-limiting Examples, which are given by way of example only.

### Example 1

A sunscreen composition comprising the following constituents was prepared:

| | % w/w |
|---|---|
| 1) Isopropyl palmitate | 0.251 |
| 2) Lecithin (sold under the trade name P Centrolex) | 0.127 |
| 3) Sorbitan sesquioleate (sold under the trade name Arlacel 83) | 3.0 |
| 4) Butylated hydroxytoluene | 0.05 |
| 5) Cetyl Dimethicone | 5.0 |
| 6) A mixture of silicone copolyol and cyclomethicone (sold under the trade designation Silicone Fluid 3225 C) | 12.0 |
| 7) Cyclomethicone (sold under the trade designation Silicone Fluid 345DC) | 15.0 |
| 8) Titanium Dioxide (sold under the trade designation MT 100T) | 8.0 |
| 9) Iron (III) Oxide (sold under the trade designation Super-Fine) | 0.111 |
| 10) Bronopol | 0.02 |
| 11) Sodium Dehydroacetate | 0.15 |
| 12) Sodium Citrate | 4.0 |
| 13) Purified Water | to 100 |

Components 1 and 2 were mixed together and heated at 75-80°C until the lecithin dissolved. Components 3 and 4 were mixed together and heated at 50°C to dissolve component 4. Components 5, 6 and 7, the mixture of components 1 and 2 and the mixture of components 3 and 4 were mixed together in a stainless steel beaker. Components 8 and 9 were added to this mixture and dispersed using a high shear mixer/homogeniser (Silverson) to produce the oil phase. Components 10, 11 and 12 were dissolved in water. This aqueous solution was added slowly to the oil phase with paddle stirring. When the addition was complete the mixture was homogenised to a suitable viscosity.

### Example 2

A sunscreen composition comprising the following constituents was prepared:

| | % w/w |
|---|---|
| 1) Isopropyl palmitate | 0.251 |
| 2) Lecithin (sold under the trade name P Centrolex) | 0.127 |
| 3) Sorbitan sesquioleate (sold under the trade name Arlacel 83) | 3.0 |
| 4) Butylated hydroxytoluene | 0.05 |
| 5) Cetyl Dimethicone | 5.0 |
| 6) A mixture of silicone copolyol and cyclomethicone (sold under the trade designation Silicone Fluid 3225 C) | 12.0 |
| 7) Cyclomethicone (sold under the trade designation Silicone Fluid 345DC) | 15.0 |
| 8) Titanium Dioxide (sold under the trade designation MT 500B) | 8.0 |
| 9) Iron (III) Oxide (sold under the trade designation Super-Fine) | 0.111 |
| 10) Bronopol | 0.02 |
| 11) Sodium Dehydroacetate | 0.15 |
| 12) Sodium Citrate | 4.0 |
| 13) Purified Water | to 100 |

### Control Example 1

This was prepared as described for Example 1 but omitting component 9.

### Control Example 2

This was prepared as described for Example 2 but omitting component 9.

### RESULTS

| **Diffey Scan (Mean of 3 readings)** | | | | |
|---|---|---|---|---|
| Wavelength (nm) | Ex. 1 | Control Ex. 1 | Ex. 2 | Control Ex. 2 |
| 290 | 13.72 | 17.57 | 14.56 | 13.49 |
| 295 | 13.99 | 17.88 | 14.97 | 13.83 |
| 300 | 13.99 | 17.94 | 15.16 | 14.04 |
| 305 | 13.84 | 17.75 | 15.25 | 14.19 |
| 310 | 13.48 | 17.24 | 15.29 | 14.27 |
| 315 | 12.94 | 16.31 | 15.15 | 14.22 |
| 320 | 12.11 | 15.27 | 14.92 | 13.96 |
| 325 | 11.23 | 14.06 | 14.48 | 13.67 |
| 330 | 10.26 | 12.66 | 13.90 | 13.19 |
| 335 | 9.13 | 11.12 | 13.07 | 12.45 |
| 340 | 8.14 | 9.68 | 12.48 | 11.82 |
| 345 | 7.24 | 8.48 | 11.72 | 11.12 |
| 350 | 6.50 | 7.46 | 10.93 | 10.25 |
| 355 | 5.81 | 6.52 | 10.06 | 9.56 |
| 360 | 5.22 | 5.80 | 9.29 | 8.79 |
| 365 | 4.76 | 5.15 | 8.53 | 8.11 |
| 370 | 4.36 | 4.66 | 7.92 | 7.47 |
| 375 | 4.01 | 4.22 | 7.31 | 6.89 |
| 380 | 3.71 | 3.82 | 6.75 | 6.35 |
| 385 | 3.44 | 3.50 | 6.20 | 5.78 |
| 390 | 3.22 | 3.22 | 5.61 | 5.26 |
| 395 | 3.05 | 3.02 | 5.25 | 4.79 |
| 400 | 2.90 | 2.87 | 4.89 | 4.44 |
| Simpson's Ratio | 0.66 | 0.64 | 0.81 | 0.81 |

These results demonstrate the efficacy of Examples 1 and 2 as sunscreens.

### Chromometer Results

| **MT 100T** | | | |
|---|---|---|---|
| Background | L = 65.93 | b = 9.76 | Mean of 10 readings |
| Control Ex.1 | L = 66.01 | b = 5.89 | |
| Example 1 | L = 65.35 | b = 8.73 | |

| **MT 500B** | | | |
|---|---|---|---|
| Background | L = 64.23 | b = 9.60 | Mean of 10 readings |
| Control Ex.2 | L = 68.05 | b = 3.69 | |
| Example 2 | L = 66.29 | b = 8.57 | |

L = measure of reflected light. The whiter the surface the higher the value.
b = measure of blue/yellow light. The lower the number the more this light is reflected.

When the control examples are applied to the skin the amount of whiteness is increased and the amount of blue light reflected is increased. When Examples 1 and 2 are applied the amount of reflected light is reduced along with the amount of blue light almost back to background levels demonstrating the advantage obtained by the incorporation of component 9 in Examples 1 and 2.

## Claims (Claims for the following Contracting State(s): DE, FR, GB, IT)

1. A sunscreen composition comprising a metal oxide which is titanium dioxide only, zinc oxide only or a mixture of both these oxides, the metal oxide being present in an amount from 0.1% to 30% by weight of the total composition, the metal oxide having a mean particle size of less than 100nm; and iron oxide present in an amount from 0.01% to 1% by weight of the total composition, the iron oxide having a mean particle size from 0.1nm to 10nm.

2. A sunscreen composition according to claim 1, comprising a water-in-oil emulsion which comprises from 5% to 70% by weight of an oil phase, from 1% to 15% by weight of an emulsifier and at least 20% by weight of an aqueous phase.

3. A sunscreen composition according to claim 1, comprising an oil-in-water emulsion which comprises from 5% to 60% by weight of an oil phase, from 1% to 20% by weight of an emulsifier and at least 35% by weight of an aqueous phase.

4. A sunscreen composition according to any preceding claim, in which the metal oxide comprises from 0.5% to 20% by weight of the total composition.

5. A sunscreen composition according to any preceding claim, in which the metal oxide comprises from 1% to 15% by weight of the total composition.

6. A sunscreen composition according to any preceding claim, in which the metal oxide comprises from 4% to 12% by weight of the total composition.

7. A sunscreen composition according to any preceding claim, in which the metal oxide is titanium dioxide.

8. A sunscreen composition according to any preceding claim, in which the metal oxide has a mean particle size of from 5nm to 50nm.

9. A sunscreen composition according to any preceding claim, in which the metal oxide has a mean particle size of from 15nm to 30nm.

10. A sunscreen composition according to any preceding claim, in which the iron oxide comprises from 0.02% to 0.5% by weight of the total composition.

11. A sunscreen composition according to any preceding claim, in which the iron oxide comprises from 0.05% to 0.25% by weight of the total composition.

12. A sunscreen composition according to any preceding claim, in which the iron oxide comprises 0.111% by weight of the total composition.

13. A sunscreen composition according to any preceding claim, in which the iron oxide has a mean particle size of from 1nm to 5nm.

14. A sunscreen composition according to any preceding claim, in which the iron oxide comprises iron (III) oxide.

15. A sunscreen composition according to any preceding claim, in which the iron oxide particles, or the metal oxide particles, or both, are substantially coated with a phospholipid.

16. A sunscreen composition according to claim 15, in which the phospholipid is lecithin.

17. A sunscreen composition according to any preceding claim in which phosphate anions are present in an amount from 0.025% to 30% by weight of the total composition.

## Claims (Claims for the following Contracting State(s): ES)

1. A method of preparing a sunscreen composition comprising mixing a metal oxide which is titanium dioxide only, zinc oxide only or a mixture of both these oxides, the metal oxide being present in an amount from 0.1% to 30% by weight of the total composition, and the metal oxide having a mean particle size of less than 100nm; with iron oxide present in an amount from 0.01% to 1% by weight of the total composition, the iron oxide having a mean particle size from 0.1nm to 10nm.

2. A method according to claim 1, in which the sunscreen composition is a water-in-oil emulsion which comprises from 5% to 70% by weight of an oil phase, from 1% to 15% by weight of an emulsifier and at least 20% by weight of an aqueous phase.

3. A method according to claim 1, in which the sunscreen composition is an oil-in-water emulsion which comprises from 5% to 60% by weight of an oil phase, from 1% to 20% by weight of an emulsifier and at least 35% by weight of an aqueous phase.

4. A method according to any preceding claim, in which the metal oxide comprises from 0.5% to 20% by weight of the total composition.

5. A method according to any preceding claim, in which the metal oxide comprises from 1% to 15% by weight of the total composition.

6. A method according to any preceding claim, in which the metal oxide comprises from 4% to 12% by weight of the total composition.

7. A method according to any preceding claim, in which the metal oxide is titanium dioxide.

8. A method according to any preceding claim, in which the metal oxide has a mean particle size of from 5nm to 50nm.

9. A method according to any preceding claim, in which the metal oxide has a mean particle size of from 15nm to 30nm.

10. A method according to any preceding claim, in which the iron oxide comprises from 0.02% to 0.5% by weight of the total composition.

11. A method according to any preceding claim, in which the iron oxide comprises from 0.05% to 0.25% by weight of the total composition.

12. A method according to any preceding claim, in which the iron oxide comprises 0.111% by weight of the total composition.

13. A method according to any preceding claim, in which the iron oxide has a mean particle size of from 1nm to 5nm.

14. A method according to any preceding claim, in which the iron oxide comprises iron (III) oxide.

15. A method according to any preceding claim, in which the iron oxide particles, or the metal oxide particles, or both, are substantially coated with a phospholipid.

16. A method according to claim 15, in which the phospholipid is lecithin.

17. A method according to any preceding claim in which phosphate anions are present in an amount from 0.025% to 30% by weight of the total composition.

18. A sunscreen composition prepared by a method according to any preceeding claim.

## Patentansprüche (Patentansprüche für folgende(n) Vertragsstaat(en): DE, FR, GB, IT)

1. Sonnenschutzmittel, das ein Metalloxid, bei dem es sich jeweils nur um Titandioxid oder Zinkoxid bzw. ein Gemisch beider Oxide handelt, wobei das Metalloxid in einer Menge von 0,1 bis 30 Gew.-% der gesamten Zubereitung vorhanden ist und eine Teilchengröße unterhalb von 100 nm aufweist, und Eisenoxid enthält, das in einer Menge von 0,01 bis 1 Gew.-% der gesamten Zubereitung vorliegt und eine mittlere Teilchengröße von 0,1 bis 10 nm aufweist.

2. Sonnenschutzmittel gemaß Anspruch 1, welches eine Wasser-in-Öl-Emulsion aufweist, die 5 bis 70 Gew.-% einer Ölphase, 1 bis 15 Gew.-% eines Emulgators und mindestens 20 Gew.-% einer wäßrigen Phase enthält.

3. Sonnenschutzmittel gemaß Anspruch 1, welches eine Wasser-in-Öl-Emulsion aufweist, die 5 bis 60 Gew.-% einer Ölphase, 1 bis 20 Gew.-% eines Emulgators und mindestens 35 Gew.-% einer wäßrigen Phase enthält.

4. Sonnenschutzmittel gemäß einem der vorstehenden Ansprüche, in dem das Metalloxid 0,5 bis 20 Gew.-% der gesamten Zubereitung ausmacht.

5. Sonnenschutzmittel gemäß einem der vorstehenden Ansprüche, in dem das Metalloxid 1 bis 15 Gew.-% der gesamten Zubereitung ausmacht.

6. Sonnenschutzmittel gemäß einem der vorstehenden Ansprüche, in dem das Metalloxid 4 bis 12 Gew.-% der gesamten Zubereitung ausmacht.

7. Sonnenschutzmittel gemäß einem der vorstehenden Ansprüche, in dem das Metalloxid Titandioxid ist.

8. Sonnenschutzmittel gemäß einem der vorstehenden Ansprüche, in dem das Metalloxid eine mittlere Teilchengröße von 5 bis 50 nm aufweist.

9. Sonnenschutzmittel gemäß einem der vorstehenden Ansprüche, in dem das Metalloxid eine mittlere Teilchengröße von 15 bis 30 nm aufweist.

10. Sonnenschutzmittel gemäß einem der vorstehenden Ansprüche, in dem das Eisenoxid 0,02 bis 0,5 Gew.-% der gesamten Zubereitung ausmacht.

11. Sonnenschutzmittel gemäß einem der vorstehenden Ansprüche, in dem das Eisenoxid 0,05 bis 0,25 Gew.-% der gesamten Zubereitung ausmacht.

12. Sonnenschutzmittel gemäß einem der vorstehenden Ansprüche, in dem das Eisenoxid 0,111 Gew.-% der gesamten Zubereitung ausmacht.

13. Sonnenschutzmittel gemäß einem der vorstehenden Ansprüche, in dem das Eisenoxid eine mittlere Teilchengröße von 1 bis 5 nm aufweist.

14. Sonnenschutzmittel gemäß einem der vorstehenden Ansprüche, in dem das Eisenoxid noch Eisen-(III)-oxid mitumfaßt.

15. Sonnenschutzmittel gemäß einem der vorstehenden Ansprüche, in dem die Eisenoxidteilchen, die Metalloxidteilchen oder beide im wesentlichen mit einem Phospholipid beschichtet sind.

16. Sonnenschutzmittel gemäß Anspruch 15, in dem das Phospholipid Lecithin ist.

17. Sonnenschutzmittel gemäß einem der vorstehenden Ansprüche, das Phosphationen in einer Menge von 0,025 bis 30 Gew.-% der gesamten Zubereitung enthält.

## Patentansprüche (Patentansprüche für folgende(n) Vertragsstaat(en): ES)

1. Verfahren zur Herstellung eines Sonnenschutzmittel, bei dem man ein Metalloxid, bei dem es sich jeweils nur um Titandioxid oder Zinkoxid bzw. ein Gemisch beider Oxide handelt, wobei das Metalloxid in einer Menge von 0,1 bis 30 Gew.-% der gesamten Zubereitung vorhanden ist und eine Teilchengröße unter 100 nm aufweist, und Eisenoxid, das in einer Menge von 0,01 bis 1 Gew.-% der gesamten Zubereitung vorliegt und eine mittlere Teilchengröße von 0,1 bis 10 nm aufweist, miteinander vermischt.

2. Verfahren gemaß Anspruch 1, bei dem das Sonnenschutzmittel eine Wasser-in-Öl-Emulsion darstellt, die 5 bis 70 Gew.-% einer Ölphase, 1 bis 15 Gew.-% eines Emulgators und mindestens 20 Gew.-% einer wäßrigen Phase enthält.

3. Verfahren gemäß Anspruch 1, bei dem das Sonnenschutzmittel eine Wasser-in-Öl-Emulsion darstellt, die 5 bis 60 Gew.-% einer Ölphase, 1 bis 20 Gew.-% eines Emulgators und mindestens 35 Gew.-% einer wäßrigen Phase enthält.

4. Verfahren gemäß einem der vorstehenden Ansprüche, bei dem das Metalloxid 0,5 bis 20 Gew.-% der gesamten Zubereitung ausmacht.

5. Verfahren gemäß einem der vorstehenden Ansprüche, bei dem das Metalloxid 1 bis 15 Gew.-% der gesamten Zubereitung ausmacht.

6. Verfahren gemäß einem der vorstehenden Ansprüche, bei dem das Metalloxid 4 bis 12 Gew.-% der gesamten Zubereitung ausmacht.

7. Verfahren gemäß einem der vorstehenden Ansprüche, bei dem das Metalloxid Titandioxid ist.

8. Verfahren gemäß einem der vorstehenden Ansprüche, bei dem das Metalloxid eine mittlere Teilchengröße von 5 bis 50 nm aufweist.

9. Verfahren gemäß einem der vorstehenden Ansprüche, bei dem das Metalloxid eine mittlere Teilchengröße von 15 bis 30 nm aufweist.

10. Verfahren gemäß einem der vorstehenden Ansprüche, bei dem das Eisenoxid 0,02 bis 0,5 Gew.-% der gesamten Zubereitung ausmacht.

11. Verfahren gemäß einem der vorstehenden Ansprüche, bei dem das Eisenoxid 0,05 bis 0,25 Gew.-% der gesamten Zubereitung ausmacht.

12. Verfahren gemäß einem der vorstehenden Ansprüche, bei dem das Eisenoxid 0,111 Gew.-% der gesamten Zubereitung ausmacht.

13. Verfahren gemäß einem der vorstehenden Ansprüche, bei dem das Eisenoxid eine mittlere Teilchengröße von 1 bis 5 nm aufweist.

14. Verfahren gemäß einem der vorstehenden Ansprüche, bei dem das Eisenoxid noch Eisen-(III)-oxid mitumfaßt.

15. Verfahren gemäß einem der vorstehenden Ansprüche, bei dem die Eisenoxidteilchen, die Metalloxidteilchen oder beide im wesentlichen mit einem Phospholipid beschichtet sind.

16. Verfahren gemäß Anspruch 15, bei dem das Phospholipid Lecithin ist.

17. Verfahren gemäß einem der vorstehenden Ansprüche, bei dem Phosphationen in einer Menge von 0,025 bis 30 Gew.-% der gesamten Zubereitung vorhanden sind.

18. Sonnenschutzmittel hergestellt nach einem Verfahren gemäß einem der vorstehenden Ansprüche.

## Revendications (Revendications pour l'(les) Etat(s) contractant(s) suivant(s): DE, FR, GB, IT)

1. Composition de protection solaire comprenant un oxyde métallique qui est du dioxyde de titane seulement, de l'oxyde de zinc seulement ou un mélange de ces deux oxydes, l'oxyde métallique étant présent dans une proportion de 0,1% à 30% en poids de la composition totale, l'oxyde métallique ayant une taille de particules moyenne inférieure à 100 nm; et de l'oxyde de fer présent dans une proportion de 0,01% à 1% en poids de la composition totale, l'oxyde de fer ayant une taille moyenne de particules de 0,1 nm à 10 nm.

2. Composition de protection solaire selon la revendication 1, comprenant une émulsion eau-dans-huile qui comprend de 5% à 70% en poids d'une phase huileuse, de 1% à 15% en poids d'un émulsionnant et au moins 20% en poids d'une phase aqueuse.

3. Composition de protection solaire selon la revendication 1, comprenant une émulsion huile-dans-eau qui comprend de 5% à 60% en poids d'une phase huileuse, de 1% à 20% en poids d'un émulsionnant et au moins 35% en poids d'une phase aqueuse.

4. Composition de protection solaire selon l'une quelconque des revendications précédentes, dans laquelle l'oxyde métallique représente de 0,5% à 20% en poids de la composition totale.

5. Composition de protection solaire selon l'une quelconque des revendications précédentes, dans laquelle l'oxyde métallique représente de 1% à 15% en poids de la composition totale.

6. Composition de protection solaire selon l'une quelconque des revendications précédentes, dans laquelle l'oxyde métallique représente de 4% à 12% en poids de la composition totale.

7. Composition de protection solaire selon l'une quelconque des revendications précédentes dans laquelle l'oxyde métallique est le dioxyde de titane.

8. Composition de protection solaire selon l'une quelconque des revendications précédentes, dans laquelle l'oxyde métallique a une taille moyenne de particules de 5 nm à 50 nm.

9. Composition de protection solaire selon l'une quelconque des revendications précédentes, dans laquelle l'oxyde métallique a une taille moyenne de particules de 15 nm à 30 nm.

10. Composition de protection solaire selon l'une quelconque des revendications précédentes, dans laquelle l'oxyde de fer représente de 0,02% à 0,5% en poids de la composition totale.

11. Composition de protection solaire selon l'une quelconque des revendications précédentes, dans laquelle l'oxyde de fer représente de 0,05% à 0.25% en poids de la composition totale.

12. Composition de protection solaire selon l'une quelconque des revendications précédentes, dans laquelle l'oxyde de fer représente 0,111% en poids de la composition totale.

13. Composition de protection solaire selon l'une quelconque des revendications précédentes, dans laquelle l'oxyde de fer a une taille moyenne de particules de 1 nm à 5 nm.

14. Composition de protection solaire selon l'une quelconque des revendications précédentes, dans laquelle l'oxyde de fer comprend de l'oxyde de fer (III).

15. Composition de protection solaire selon l'une quelconque des revendications précédentes, dans laquelle les particules d'oxyde de fer ou les particules d'oxyde métallique, ou les deux, sont revêtues en grande partie d'un phospholipide.

16. Composition de protection solaire selon la revendication 15, dans laquelle le phospholipide est la lécithine.

17. Composition de protection solaire selon l'une quelconque des revendications précédentes, dans laquelle des anions phosphate sont présents dans une proportion de 0,025% à 30% en poids de la composition totale.

## Revendications (Revendications pour l'(les) Etat(s) contractant(s) suivant(s): ES)

1. Procédé de préparation d'une composition de protection solaire consistant à mélanger un oxyde métallique qui est du dioxyde de titane seulement, de l'oxyde de zinc seulement ou un mélange de ces deux oxydes, l'oxyde métallique étant présent dans une proportion de 0,1% à 30% en poids de la composition totale, et l'oxyde métallique ayant une taille moyenne de particules inférieure à 100 nm; de l'oxyde de fer étant présent dans une proportion de 0,01% à 1% en poids de la composition totale, l'oxyde de fer ayant une taille moyenne de particules de 0,1 nm à 10 nm.

2. Procédé selon la revendication 1, dans lequel la composition de protection solaire est une émulsion eau-dans-huile qui comprend de 5% à 70% en poids d'une phase huileuse, de 1% à 15% en poids d'un émulsionnant et au moins 20% en poids d'une phase aqueuse.

3. Procédé selon la revendication 1, dans lequel la composition de protection solaire est une émulsion huile-dans-eau qui comprend de 5% à 60% en poids d'une phase huileuse, de 1% à 20% en poids d'un émulsionnant et au moins 35% en poids d'une phase aqueuse.

4. Procédé selon l'une quelconque des revendications précédentes, dans lequel l'oxyde métallique constitue de 0,5% à 20% en poids de la composition totale.

5. Procédé selon l'une quelconque des revendications précédentes, dans lequel l'oxyde métallique représente de 1% à 15% en poids de la composition totale.

6. Procédé selon l'une quelconque des revendications précédentes, dans lequel l'oxyde métallique représente de 4% à 12% en poids de la composition totale.

7. Procédé selon l'une quelconque des revendications précédentes, dans lequel l'oxyde métallique est le dioxyde de titane.

8. Procédé selon l'une quelconque des revendications précédentes, dans lequel l'oxyde métallique a une taille moyenne de particules de 5 nm à 50 nm.

9. Procédé selon l'une quelconque des revendications précédentes, dans lequel l'oxyde métallique a une taille moyenne de particules de 15 nm à 30 nm.

10. Procédé selon l'une quelconque des revendications précédentes, dans lequel l'oxyde de fer constitue de 0,02% à 0,5% en poids de la composition totale.

11. Procédé selon l'une quelconque des revendications précédentes, dans lequel l'oxyde de fer représente de 0,05% à 0,25% en poids de la composition totale.

12. Procédé selon l'une quelconque des revendications précédentes, dans lequel l'oxyde de fer représente 0,111% en poids de la composition totale.

13. Procédé selon l'une quelconque des revendications précédentes, dans lequel l'oxyde de fer a une taille moyenne de particules de 1 nm à 5 nm.

14. Procédé selon l'une quelconque des revendications précédentes, dans lequel l'oxyde de fer comprend de l'oxyde de fer (III).

15. Procédé selon l'une quelconque des revendications précédentes, dans lequel les particules d'oxyde de fer ou les particules d'oxyde métallique, ou les deux, sont en grande partie revêtues d'un phospholipide.

16. Procédé selon la revendication 15, dans lequel le phospholipide est la lécithine.

17. Procédé selon l'une quelconque des revendications précédentes, dans lequel des anions phosphate sont présents dans une proportion de 0,025% à 30% en poids de la composition totale.

18. Composition de protection solaire préparée par un procédé selon l'une quelconque des revendications précédentes.
